# EUROPEAN PATENT APPLICATION

(11) **EP 2 638 843 A1**
(43) Date of publication of application: **18.09.2013**
(21) Application number: 13156822.2
(22) Date of filing: 26.02.2013
(51) Int. Cl.: A61B 1/00, A61B 1/045, A61B 1/06, G06T 7/00

(54) **Endoscope system, processor device thereof, and exposure control method**

(30) Priority: 14.03.2012 JP 2012057284
(71) Applicant: Fujifilm Corporation, Minato-ku Tokyo (JP)
(72) Inventor: Saito, Takaaki, Kanagawa (JP)
(74) Representative: Klunker . Schmitt-Nilson . Hirsch

(57) **Abstract**

When an endoscope system (10) is put into a special mode, first and second frame periods for performing imaging under first and second measurement light to measure an oxygen saturation level, a third frame period for performing imaging under normal light, and a fourth frame period for performing imaging under vessel detection light to detect blood vessels in specific depth are repeated. An oxygen saturation image, a normal image, and a vessel pattern image are produced and displayed in a tiled manner on a monitor (14) in the form of moving images. When a freeze button (16b) is pressed during display of the moving images, the light intensity and exposure time to be used in the first to fourth frame periods of a still image recording process are calculated using an image that is captured immediately before pressing the freeze button (16b). Still images are obtained with the calculated light intensity and exposure time.

## Description

### BACKGROUND OF THE INVENTION

### 1. Field of the Invention

The present invention relates to an endoscope system that produces not only a normal image captured under normal light such as white light, but also a special image captured under special light in a specific narrow wavelength band, a processor device of the endoscope system, and an exposure control method.

### 2. Description Related to the Prior Art

An endoscope system is widely used in examination of the interior of a patient's body, e.g. a digestive system. The endoscope system is constituted of an electronic endoscope to be introduced into the patient's body, a light source device for supplying illumination light to the electronic endoscope, a processor device for processing an image (moving image) captured by the electronic endoscope, and a monitor for displaying the image after being processed by the processor device. In recent years, there is known an endoscope system that carries out not only normal observation for imaging an internal body portion under white light (normal light) but also special observation for imaging the internal body portion irradiated with specific narrowband light (special light).

As the special observation, a blood vessel pattern obtaining technique is known in which a blood vessel in a specific depth is emphasized by applying the special light that has a wavelength having a high light absorption coefficient of hemoglobin. Whether or not an obtained blood vessel pattern matches with a pattern specific to cancer is judged to find out a cancer-suspected lesion.

Also, there is known an oxygen saturation level obtaining technique. This technique uses first illumination light being narrowband light in a wavelength band at which a light absorption coefficient differs between oxygenated hemoglobin and deoxygenated hemoglobin, and second illumination light in a wavelength band different from that of the first illumination light. Sequentially applying the first and second illumination light to the internal body portion allows obtainment of an oxygen saturation level of blood. According to this technique, a hypoxic area being a cancer symptom is indicated by artificial color, so it is possible to find out cancer at sight.

To accurately find out cancer, a normal moving image obtained in the normal observation and two types of special moving images, that is, a vessel pattern moving image and an oxygen saturation moving image obtained in the special observation are preferably displayed together in a single monitor. Simultaneously displaying the plurality of types of moving images, as described above, facilitates making a diagnosis from various viewpoints, and hence greatly improving diagnostic accuracy. Note that, Japanese Patent Laid-Open Publication No. 2003-033324 discloses simultaneous display of a plurality of types of moving images in detail.

During the display of the moving images, a frame of each moving image that captures a lesion is recorded as a still image upon a press of a freeze button provided in the electronic endoscope. The recorded still images of a plurality of types are used as material for explaining to the patient about his/her physical condition and as material for discussion among doctors.

As described above, the normal moving image, the vessel pattern moving image, and the oxygen saturation moving image are produced using the plurality of types of illumination light in the wavelength bands different from one another. Thus, in order to obtain and display the plurality of types of moving images at the same time, lighting is sequentially switched to take out the illumination light required for producing each moving image, and imaging is performed in synchronization with the switching of lighting. The amount of illumination light required for appropriate exposure differs from one moving image to another, so it is necessary to perform exposure control in accordance with each moving image. In general, the exposure control is performed based on the brightness of an immediately preceding frame image. However, in the case of switching and applying the plurality of types of illumination light in a sequential manner, the type of image to be subjected to the exposure control is different from the type of immediately preceding frame image. Therefore, the exposure control cannot be performed appropriately.

### SUMMARY OF THE INVENTION

An object of the present invention is to provide an endoscope system in which when a still image is taken out from each of a plurality of types of moving images in the middle of capturing the moving images, exposure strength is controlled appropriately in accordance with the type of each still image, a processor device of the endoscope system, and a method therefor.

To achieve the above and other objects of the present invention, an endoscope system according to the present invention includes a lighting means, an image pickup means, a moving image processing means, a monitor means, an exposure condition determining means, an exposure control means, and a still image recording means. The lighting means produces a plurality of types of illumination light having different wavelength bands sequentially in accordance with a plurality of types of frame periods, and applies the illumination light to an internal body portion. The image pickup means has an image sensor for imaging the internal body portion. The image pickup means sequentially captures a plurality of types of frame images. The moving image processing means produces a plurality of types of moving images, corresponding to the plurality of types of illumination light, from the plurality of types of frame images sequentially captured by the image pickup means. The monitor means displays the plurality of types of moving images. The exposure condition determining means determines an exposure condition of each of a plurality of types of still images to be recorded in a still image recording process in accordance with the types of frame periods, with use of one of the frame images captured immediately before or immediately after a start operation of the still image recording process as a reference image. The exposure control means controls exposure in accordance with the exposure condition determined independently from one type of frame period to another. The still image recording means takes out the frame images captured under control of the exposure as the still images, to perform the still image recording process.

The exposure condition determining means preferably includes a memory and a calculator. The memory stores correlation in the exposure condition among the plurality of types of frame images. The calculator calculates the exposure condition to be used in capturing each of the still images based on the reference image and the correlation.

The correlation is preferably a light intensity ratio among the plurality of types of illumination light applied in the frame periods, or an exposure time ratio of the image sensor among the frame periods.

When the plurality of types of still images obtained in a sequence is referred to as a frame set, the still image recording means preferably obtains a plurality of frame sets, and calculates positional deviation among the still images in each frame set, and records to still image storage the frame set whose positional deviation is less than a predetermined threshold value.

After the still image recording process, all of the recorded still images are preferably displayed on the monitor means in a tiled manner or in succession at regular intervals.

The plurality of types of frame images may include a normal image, an oxygen saturation image, and a vessel pattern image. The normal image is obtained under irradiation with white light. The oxygen saturation image is obtained under irradiation with measurement light having a wavelength at which a light absorption coefficient much differs between oxygenated hemoglobin and deoxygenated hemoglobin. The vessel pattern image is obtained under irradiation with vessel detection light that has a wavelength having a high light absorption coefficient of hemoglobin and emphasizes a blood vessel of specific depth.

The lighting means may include a plurality of semiconductor light sources for emitting the plurality of types of illumination light, or include a white light source and wavelength selective filters. The white light source emits broadband light. Each wavelength selective filter takes out light of a specific wavelength from the broadband light.

A processor device of an endoscope system includes a moving image processing means, an exposure condition determining means, an exposure control means, and a still image recording means. The moving image processing means produces a plurality of types of moving images, corresponding to the plurality of types of illumination light, from the plurality of types of frame images sequentially captured by the image sensor. The exposure condition determining means determines an exposure condition of each of a plurality of types of still images to be recorded in a still image recording process in accordance with the types of frame periods, with use of one of the frame images captured immediately before or immediately after a start operation of the still image recording process as a reference image. The exposure control means controls exposure in accordance with the exposure condition determined independently from one type of frame period to another. The still image recording means takes out the frame images captured under control of the exposure as the still images, to perform the still image recording process.

An exposure control method of an endoscope system includes the steps of producing a plurality of types of illumination light having different wavelength bands sequentially in accordance with a plurality of types of frame periods, and applying the illumination light to an internal body portion; capturing a plurality of types of frame images sequentially with use of an image sensor for imaging the internal body portion; using as a reference image one of the frame images captured immediately before or immediately after a start operation of a still image recording process, when the start operation is performed during production of a plurality of types of moving images, corresponding to the plurality of types of illumination light, from the plurality of types of frame images sequentially captured by the image sensor; determining an exposure condition of each of a plurality of types of still images to be recorded in the still image recording process in accordance with the types of frame periods, with use of the reference image; and controlling exposure in accordance with the exposure condition determined independently from one of the types of frame periods to another.

According to the present invention, when the plurality of types of still images are recorded upon a press of a freeze button or the like, a reference exposure condition is calculated based on the reference image that is obtained immediately before or immediately after the press of the freeze button. In capturing each still image, the reference exposure condition is modified in accordance with the type of still image, and exposure strength is controlled based on the modified exposure condition. Therefore, the exposure strength is appropriately and easily adjusted in accordance with each still image.

### BRIEF DESCRIPTION OF THE DRAWINGS

For more complete understanding of the present invention, and the advantage thereof, reference is now made to the subsequent descriptions taken in conjunction with the accompanying drawings, in which:
Fig. 1 is a schematic view of an endoscope system;
Fig. 2 is a block diagram of an endoscope system according to a first embodiment;
Fig. 3A is a graph showing emission spectrum of normal light;
Fig. 3B shows graphs that represent emission spectra of first and second measurement light, the normal light, and pattern detection light;
Fig. 4A is an explanatory view of an arrangement of B, G, and R pixels in a color image sensor;
Fig. 4B is a graph showing spectral transmittance of the B, G, and R pixels;
Fig. 4C is a graph for explaining the operation of an electronic shutter;
Fig. 5A is an explanatory view of the operation of the image sensor in a normal mode of the first embodiment;
Fig. 5B is an explanatory view of the operation of the image sensor in a special mode of the first embodiment;
Fig. 6 is a block diagram of a moving image processing section;
Fig. 7 is a graph showing the correlation between an oxygen saturation level and signal ratios B1/G2 and R2/G2;
Fig. 8 is a graph showing a light absorption coefficient of hemoglobin;
Fig. 9 is a graph showing the correlation between blood volume and the signal ratio R2/G2;
Fig. 10 is an explanatory view of a method for calculating the oxygen saturation level from the signal ratios in the graph of Fig. 7;
Fig. 11A is an explanatory view of a method for producing a first vessel pattern image;
Fig. 11B is an explanatory view of a method for producing a second vessel pattern image;
Fig. 12A is a plan view of a monitor in which three types of moving images are displayed at the same time;
Fig. 12B is a plan view of the monitor in which the three types of moving images are displayed at the same time in a way different from that of Fig. 12A;
Fig. 13 is a block diagram of a still image processing section;
Fig. 14 is an explanatory view for explaining emission timing of each type of illumination light in a still image recording process;
Fig. 15 is an explanatory view for explaining imaging operation of each frame period in the still image recording process;
Fig. 16A is a plan view of the monitor in which an oxygen saturation image, a normal image, and a vessel pattern image are displayed as still images in a tiled manner;
Fig. 16B is an explanatory view for explaining a state of sequentially displaying the oxygen saturation image, the normal image, and the vessel pattern image on the monitor;
Fig. 17 is a flowchart of the special mode;
Fig. 18 is an explanatory view of an example in which two frame sets are carried out in the still image recording process;
Fig. 19 is an explanatory view of an example in which predetermined first and second light intensity ratios are used in the still image recording process;
Fig. 20 is a block diagram of an endoscope system according to a second embodiment;
Fig. 21 is a plan view of a rotary filter unit;
Fig. 22 is a graph showing spectral transmittance of each filter of the rotary filter unit;
Fig. 23A is an explanatory view of imaging control in a normal mode of the second embodiment; and
Fig. 23B is an explanatory view of imaging control in a special mode of the second embodiment.

### DESCRIPTION OF THE PREFERRED EMBODIMENTS

### (First Embodiment)

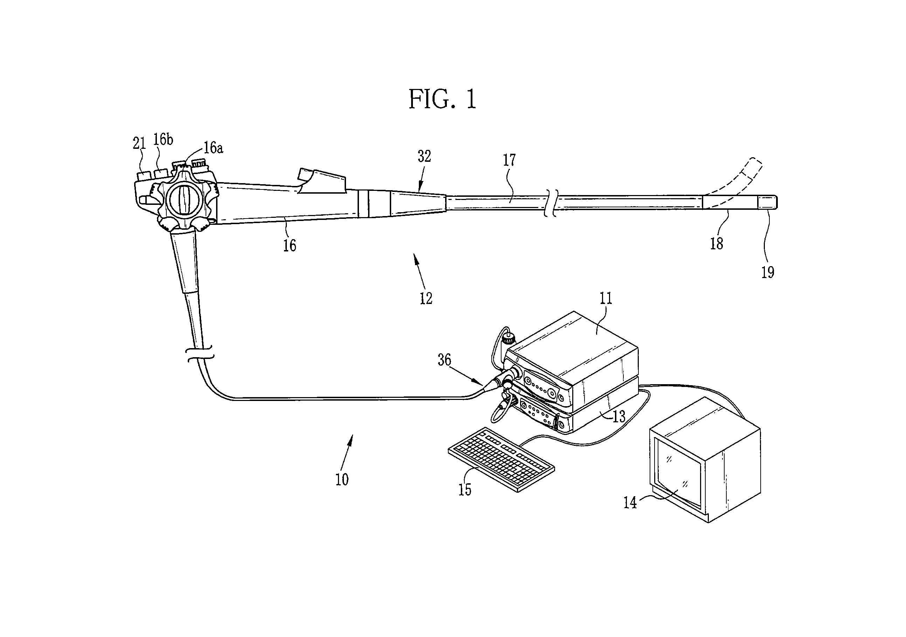

As shown in Fig. 1, an endoscope system 10 is constituted of a light source device 11, an electronic endoscope 12, a processor device 13, a monitor 14, and an input device 15 such as a keyboard. The light source device 11 produces illumination light for illuminating the interior of a patient's body cavity. The electronic endoscope 12 applies the illumination light supplied by the light source device 11 to an internal body portion, and images the body portion. The processor device 13 applies image processing to an image signal obtained by the electronic endoscope 12. Endoscopic images obtained by the image processing are sequentially displayed on the monitor 14 as a moving image.

The electronic endoscope 12 is provided with a control handle unit 16 and an insert section, which has a flexible elongated tube 17, a steering assembly 18, and a head assembly 19. The slender flexible elongated tube 17 is made of a coiled pipe, and is bent in accordance with the shape of a path in the body cavity. The steering assembly 18 is flexibly bent by a turn of an angle knob 16a provided on the control handle unit 16. By bending the steering assembly 18 in an arbitrary direction and angle, the head assembly 19 is aimed at the desired internal body portion to be examined. The control handle unit 16 is provided with a freeze button 16b, which is used in recording an endoscopic image as a still image to still image storage 75a. Upon a press of the freeze button 16b during the display of the moving image, a still image recording process is started in which a frame of the moving image is taken out and recorded as the still image. After the completion of the still image recording process, the moving image is displayed again on the monitor 14. In another case, the captured still image may be displayed on the monitor 14, and the display may be switched to the moving image upon operation of a switch button.

The endoscope system 10 is switchable between a normal mode and a special mode. In the normal mode, a normal moving image composed of a plurality of normal images is produced in the visible light range from blue to red under application of white illumination light (normal light). The produced normal moving image is displayed on the monitor 14. In the special mode, three types of moving images each composed of the normal images, vessel pattern images, and oxygen saturation images are produced. The produced three types of moving images are displayed on the monitor 14 in a tiled manner. The switching between the normal mode and the special mode is appropriately performed based on input from a mode switch 21 of the electronic endoscope 12 or the input device 15.

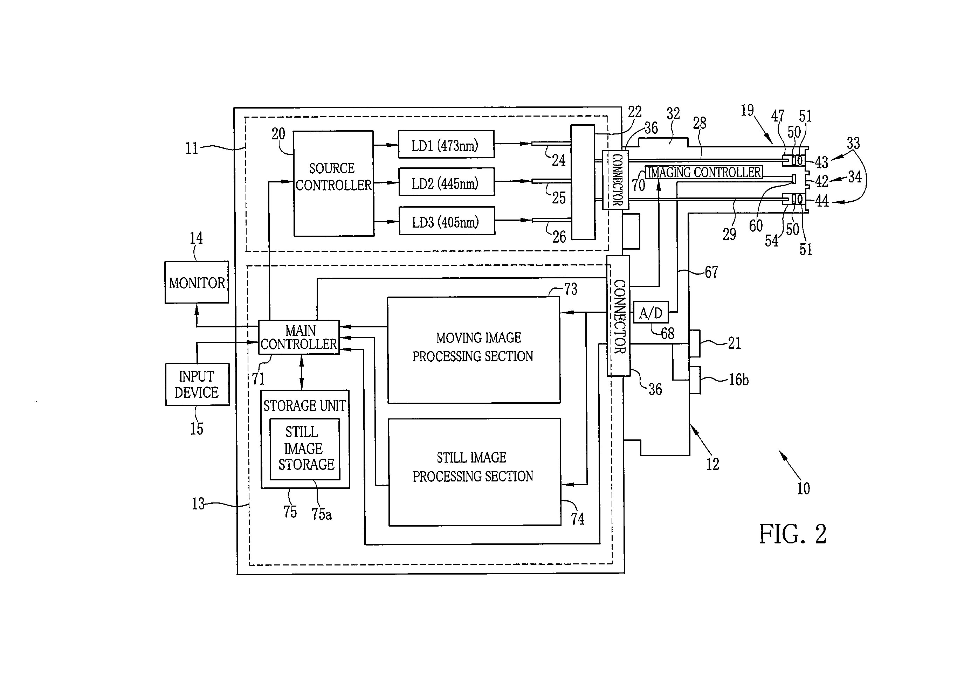

As shown in Fig. 2, the light source device 11 includes three laser sources LD1, LD2, and LD3 and a source controller 20. The laser source LD1 emits a first laser beam having a center wavelength of 473 nm. The first laser beam excites a phosphor 50 disposed in the head assembly 19 of the electronic endoscope 12, and the excited phosphor 50 produces fluorescence in a wavelength range from green to red. The laser source LD2 emits a second laser beam having a center wavelength of 445 nm. The second laser beam also excites the phosphor 50, so the excited phosphor 50 produces the fluorescence. Most of the first and second laser beams is subjected to wavelength conversion by the phosphor 50, but a part of the first and second laser beams passes through the phosphor 50. The laser source LD3 emits a third laser beam having a center wavelength of 405 nm. Although a part of the third laser beam excites the phosphor 50 and produces the fluorescence, most of the third laser beam passes through the phosphor 50. The first to third laser beams emitted from the laser sources LD1 to LD3 enter optical fibers 24 to 26 through condenser lenses (not shown), respectively.

Note that, the first laser beam is preferably in a wavelength range of 460 to 480 nm. The second laser beam is preferably in a wavelength range of 440 to 460 nm. The third laser beam is preferably in a wavelength range of 400 to 410 nm. As the laser source LD1, LD2, or LD3, a broad-area type InGaN laser diode, InGaNAs laser diode, GaNAs laser diode, or the like is available.

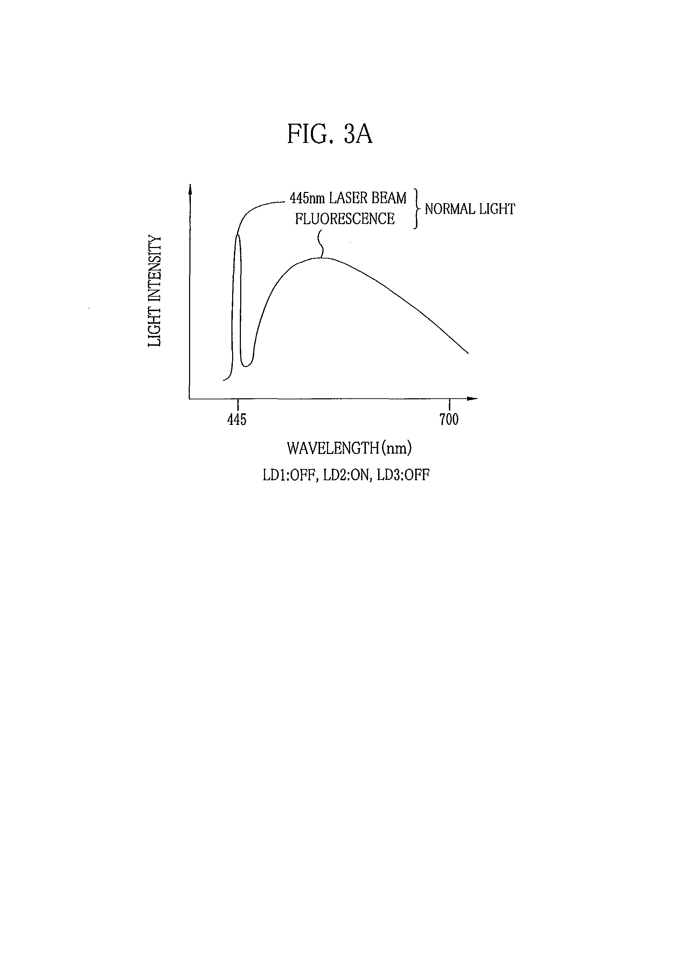

The source controller 20 controls emission timing of each of the laser sources LD1, LD2, and LD3. In the normal mode, the laser source LD2 is turned on, while the other laser sources LD1 and LD3 are turned off. Thus, the normal light having a spectrum of Fig. 3A, which includes the second laser beam from the laser source LD2 and the fluorescence from the phosphor 50 excited by the second laser beam, is applied to the internal body portion.

In the special mode, on the other hand, emission timing of each of the laser sources LD1 to LD3 differs among first and second frame periods for obtaining the oxygen saturation image, a third frame period for obtaining the normal image, and a fourth frame period for obtaining the vessel pattern image. Note that, in the special mode, the first to fourth frame periods are carried out in numerical order.

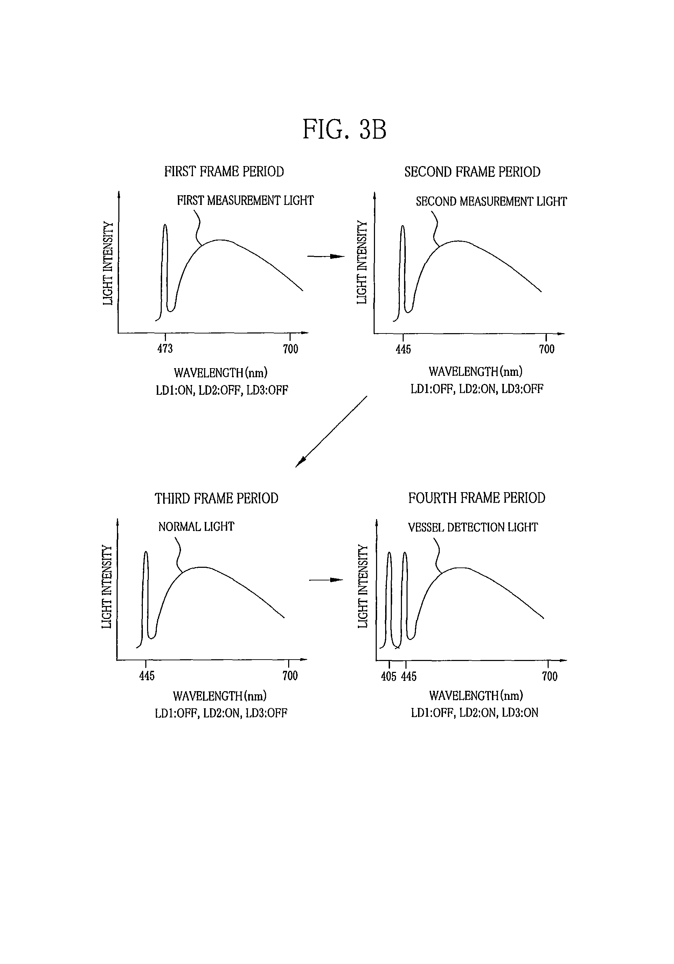

As shown in Fig. 3B, in the first frame period, the laser source LD1 is turned on, while the other laser sources LD2 and LD3 are turned off. In the second frame period, the laser source LD2 is turned on, while the other laser sources LD1 and LD3 are turned off. Thus, in the first frame period, first oxygen saturation level measurement light (hereinafter called first measurement light), which includes the first laser beam from the laser source LD1 and the fluorescence from the phosphor 50 excited by the first laser beam, is applied to the internal body portion. In the second frame period, second oxygen saturation level measurement light (hereinafter called second measurement light), which includes the second laser beam from the laser source LD2 and the fluorescence from the phosphor 50 excited by the second laser beam, is applied to the internal body portion.

In the third frame period for obtaining the normal image, as in the case of the normal mode, the normal light, which includes the second laser beam from the laser source LD2 and the fluorescence from the phosphor 50 excited by the second laser beam, is applied to the internal body portion. In the fourth frame for obtaining the vessel pattern image, the laser sources LD2 and LD3 are turned on, while the other laser source LD1 is turned off. Thus, blood vessel pattern detection light (hereinafter called vessel detection light), which includes the second laser beam from the laser source LD2, the third laser beam from the laser source LD3, and the fluorescence from the phosphor 50 excited by the second and third laser beams, is applied to the internal body portion.

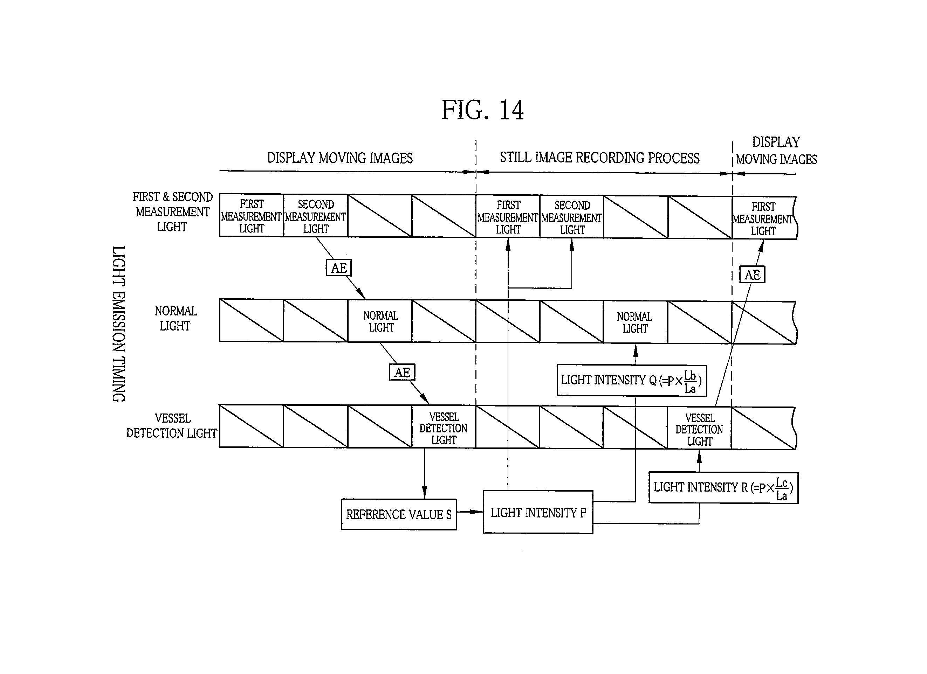

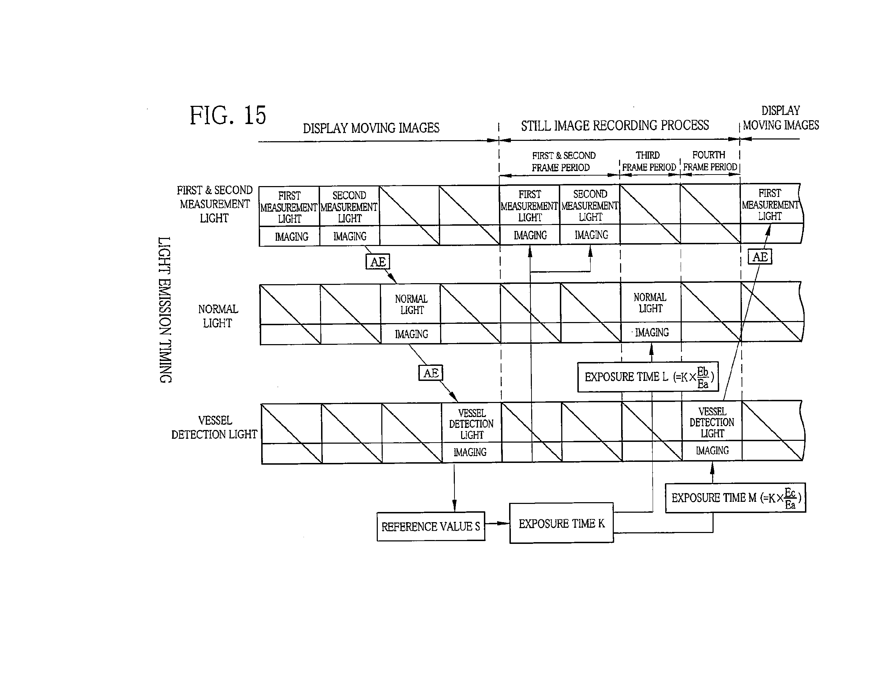

The source controller 20 controls the light intensity of each of the laser sources LD1 to LD3 under control of the processor device 13. In the normal mode, the light intensity control is performed based on an image that is obtained immediately precedently. In the special mode, on the other hand, during the display of the moving images, the light intensity control is performed based on an image that is obtained immediately precedently. During the still image recording process, the light intensity control is performed based on an image that is captured immediately before a press of the freeze button 16b (see Figs. 14 and 15).

A coupler 22 branches the first to third laser beams transmitted through the optical fibers 24 to 26 in two beams. The branched two beams are transmitted through light guides 28 and 29, respectively. Each of the light guides 28 and 29 is made of a bundle of a number of optical fibers.

As shown in Fig. 2, the electronic endoscope 12 is provided with a lighting section 33 for applying the two-system (two beams) illumination light transmitted through the light guides 28 and 29 to the internal body portion, a single-system image pickup section 34 for imaging the internal body portion, and a connector 36 through which the electronic endoscope 12 is detachably connected to the light source device 11 and the processor device 13.

The lighting section 33 includes two lighting windows 43 and 44 disposed on both sides of the image pickup section 34. In the recess of the lighting windows 43 and 44, light projection units 47 and 54 are disposed, respectively. In each of the light projection units 47 and 54, at least one of the first to third laser beams transmitted through the light guide 28, 29 enters the phosphor 50.

The phosphor 50 is made of a plurality of types of fluorescent substances (for example, YAG-based fluorescent substance or BAM (BaMgAl₁₀O₁₇)-based fluorescent substance) that absorb a part of the laser beam and emit the green to red fluorescence. The entrance of the laser beam into the phosphor 50 produces pseudo white light by mixing of the green to red fluorescence produced by the phosphor 50 excited by the laser beam and the laser beam passed through the phosphor 50 without being absorbed.

The phosphor 50 preferably has an approximately rectangular parallelepiped shape. The phosphor 50 may be formed by compacting the fluorescent substances by a binder into the rectangular parallelepiped shape. The mixture of the fluorescent substances and resin such as inorganic glass may be formed into the rectangular parallelepiped shape. This phosphor 50 is known under the trademark of Micro White (MW).

The image pickup section 34 is provided with an imaging window 42 disposed in the head assembly 19. In the recess of the imaging window 42, there is provided an imaging optical system (not shown) on which image light of the internal body portion is incident. Behind the imaging optical system, an image sensor 60 such as a CCD (charge coupled device) image sensor is provided to perform photoelectric conversion of the image light. Note that, a IT (interline transfer) type color CCD image sensor is used as the image sensor 60, but a CMOS (complementary metal-oxide semiconductor) image sensor having a global shutter function may be used instead.

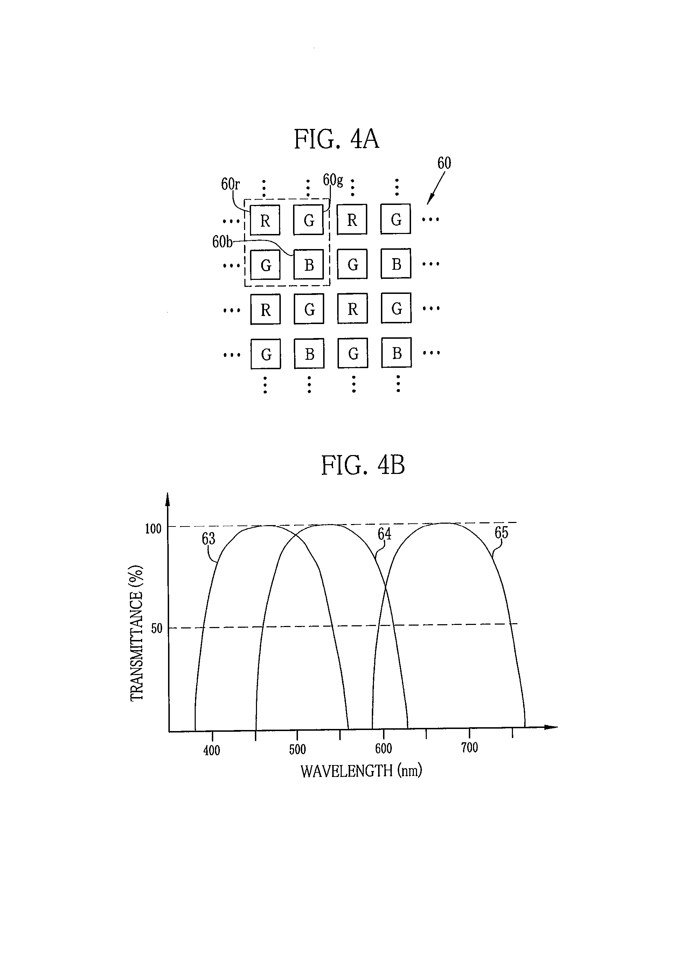

The image sensor 60 receives the image light from the internal body portion through an objective lens unit at its light receiving surface (imaging surface). The image sensor 60 performs the photoelectric conversion of the received image light, and outputs an analog image signal. As shown in Fig. 4A, the imaging surface of the image sensor 60 has 2- by 2-pixel groups arranged into matrix. Each pixel group consists of one B pixel 60b having a B (blue) color filter, two G pixels 60g having a G (green) color filter, and one R pixel 60r having a R (red) color filter. The B, G, and R color filters have high spectral transmittance in a blue wavelength range, a green wavelength range, and a red wavelength range, as represented by curves 63, 64, and 65 of Fig. 4B, respectively.

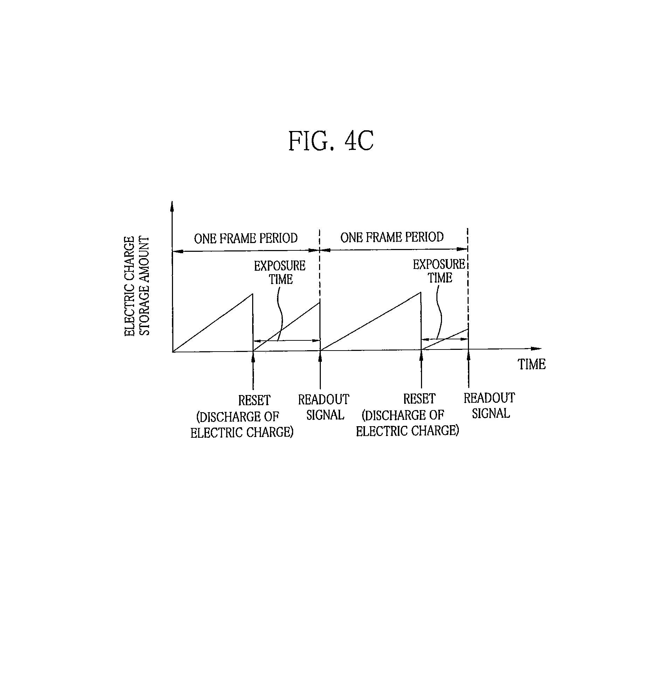

The image sensor 60 has an electronic shutter function for regulating charge storage time. By the electronic shutter function, as shown in Fig. 4C, stored electric charge is reset (discharged) at predetermined timing in one frame period. Only electric charge stored after the reset is read out as an image signal. The charge storage time after the reset corresponds to exposure time. Thus, control of the reset timing allows control of the charge storage time, and hence regulating the exposure time of the image sensor 60.

As shown in Fig. 2, the analog image signal outputted from the image sensor 60 is inputted to an A/D converter (A/D) 68 through a cable 67. The A/D 68 converts the image signal into a digital image signal in accordance with its voltage level. The converted image signal is inputted to the processor device 13 through the connector 36.

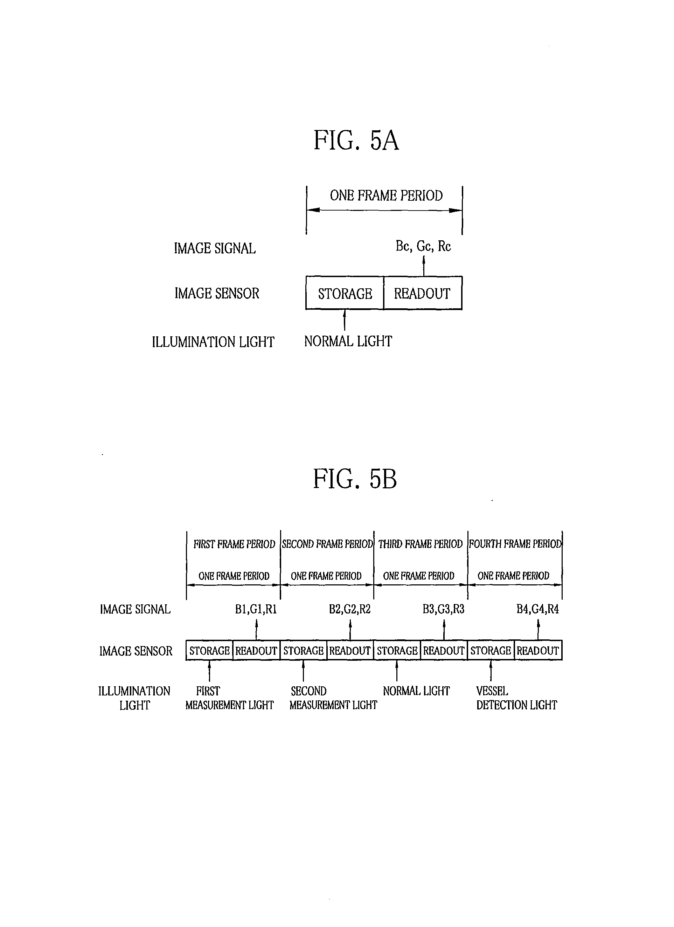

An imaging controller 70 controls the image sensor 60. As shown in Fig. 5A, in the normal mode, a storage step and a readout step are performed within one frame period. In the storage step, electric charge produced in each color pixel by the normal light is stored. In the readout step, the stored electric charge is read out as a blue signal Bc, a green signal Gc, and a red signal Rc. The storage step and the readout step are repeated, while the endoscope system 10 is in the normal mode.

In the special mode, on the other hand, as shown in Fig. 5B, in the first frame period, each color pixel stores electric charge produced by the first measurement light (first laser beam (473 nm) + fluorescence) and reads out the electric charge. A blue signal B1, a green signal G1, and a red signal R1 are read out from the color pixels. In the second frame period, electric charge produced by the second measurement light (second laser beam (445 nm) + fluorescence) is stored and read out. A blue signal B2, a green signal G2, and a red signal R2 are read out from the color pixels.

In the next third frame period, electric charge produced by the normal light is stored and read out. A blue signal B3, a green signal G3, and a red signal R3 are read out from the color pixels. In the fourth frame period, electric charge produced by the vessel detection light is stored and readout. A blue signal B4, a green signal G4, and a red signal R4 are outputted. The above first to fourth frame periods are repeated, while the endoscope system 10 is in the special mode.

As shown in Fig. 2, the processor device 13 is provided with a main controller 71, a moving image processing section 71, a still image processing section, and a storage unit 75. The main controller 71 is connected to the monitor 14 and the input device 15. The main controller 71 controls the operation of the moving image processing section 73, the source controller 20 of the light source device 11, the imaging controller 70 of the electronic endoscope 12, and the monitor 14 based on input from the mode switch 21 of the electronic endoscope 12 and the input device 15.

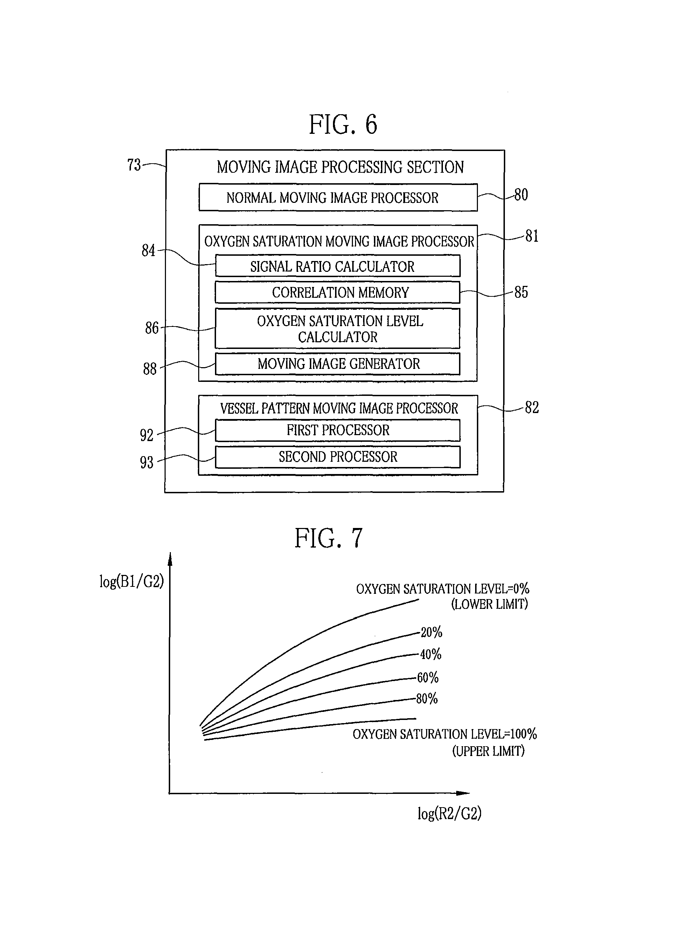

As shown in Fig. 6, the moving image processing section 73 includes a normal moving image processor 80, an oxygen saturation moving image processor 81, and a vessel pattern moving image processor 82, each of which applies predetermined image processing to the image signal from the electronic endoscope 12. In the normal mode, the normal moving image processor 80 applies the predetermined image processing to the image signals Bc, Gc, and Rc of each frame transmitted from the electronic endoscope 12, to produce the normal images in series. The plurality of normal images produced in series compose the normal moving image. In the special mode, on the other hand, the normal moving image processor 80 produces the normal images in series from the image signals B3, G3, and R3 of each frame, and a series of normal images composes the normal moving image.

The oxygen saturation moving image processor 81 calculates an oxygen saturation level of blood based on the image signals inputted in the first and second frame periods. The oxygen saturation moving image processor 81 produces an oxygen saturation image in which the normal image is artificially colored in accordance with the degree of the oxygen saturation level. A series of oxygen saturation images produces the oxygen saturation moving image. To calculate the oxygen saturation level, the oxygen saturation moving image processor 81 uses the blue signal B1, the green signal G2, and the red signal R2 out of the signals obtained in the first and second frame periods.

The oxygen saturation moving image processor 81 includes a signal ratio calculator 84, a correlation memory 85, an oxygen saturation level calculator 86, and a moving image generator 88. The signal ratio calculator 84 calculates a signal ratio B1/G2 between the blue signal B1 of the first frame period and the green signal G2 of the second frame period, and a signal ratio R2/G2 between the red signal R2 and the green signal G2 of the second frame period. The signal ratio calculator 84 calculates the signal ratios with respect to the pixel situated in the same position. The signal ratios may be calculated with respect to each and every pixel, or only in pixels situated within a blood vessel area. In this case, the blood vessel area is determined based on difference in the image signal between the blood vessel area and the other area.

The correlation memory 85 stores the correlation among the signal ratios B1/G2 and R2/G2 and the oxygen saturation level. As shown in Fig. 7, this correlation takes the form of a two-dimensional table in which contour lines representing the oxygen saturation level are defined in two-dimensional space. The position and shape of the contour lines are obtained by physical simulation of light scattering, and are variable in accordance with blood volume. For example, variation in the blood volume widens or narrows distance between the contour lines. Note that, the signal ratios B1/G2 and R2/G2 are depicted in log scale.

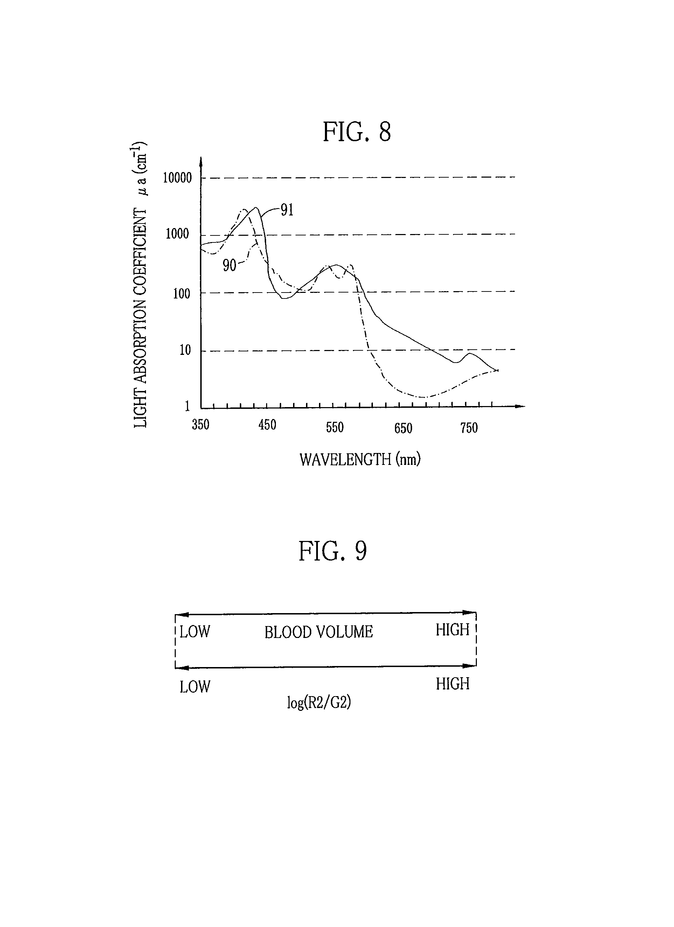

The correlation is closely related to the light absorbing property and light scattering property of oxygenated hemoglobin and deoxygenated hemoglobin, as shown in Fig. 8. In Fig. 8, a line 90 represents a light absorption coefficient of the oxygenated hemoglobin, and a line 91 represents a light absorption coefficient of the deoxygenated hemoglobin. The use of a wavelength of, for example, 473 nm at which the light absorption coefficient much differs between the oxygenated hemoglobin and the deoxygenated hemoglobin allows the obtainment of the oxygen saturation level. However, the blue signal B1 that corresponds to the light of 473 nm is highly dependent not only on the oxygen saturation level but also on the blood volume. Therefore, the use of the signal ratios B1/G2 and R2/G2, which are obtained from the red signal R2 that is mainly dependent on the blood volume and the green signal G2 being a reference signal (standardization signal) of the blue signal B1 and the red signal R2, in addition to the blue signal B1, allows the obtainment of the oxygen saturation level with high accuracy while eliminating the influence of the blood volume.

The correlation memory 85 also stores the correlation between the signal ratio R2/G2 and the blood volume as shown in Fig. 9. This correlation takes the form of a one-dimensional table in which the blood volume is increased with increase in the signal ratio R2/G2. The correlation between the signal ratio R2/G2 and the blood volume is used in calculation of the blood volume.

The following three items hold true according to the dependence of the light absorption coefficient of hemoglobin on a wavelength:
(1) In the vicinity of a wavelength of 470 nm (for example, the blue wavelength range having a center wavelength of 470 nm ± 10 nm), the light absorption coefficient largely varies in accordance with difference in the oxygen saturation level.
(2) In the green wavelength range between 540 and 580 nm, a mean value of the light absorption coefficient is insusceptible to the oxygen saturation level.
(3) In the red wavelength range between 590 and 700 nm, the light absorption coefficient seems to vary largely in accordance with the oxygen saturation level, but in actual fact, is insusceptible to the oxygen saturation level because a value of the light absorption coefficient is very small.

The reason why the signal ratio B1/G2 increases with increase in the signal ratio R2/G2, in other words, why the contour line representing the oxygen saturation level of 0% ascends slantly, as shown in Fig. 7, is as follows. As described above, the blood volume increases with increase in the signal ratio R2/G2, because of the correlation between the signal ratio R2/G2 and the blood volume. Out of the signals B1, G2, and R2, a signal value of the green signal G2 decreases most greatly with increase in the blood volume, and a signal value of the blue signal B1 decreases next greatly. This is because the light absorption coefficient is higher at a wavelength range of 540 to 580 nm included in the green signal G2 than that at a wavelength range of around 470 nm included in the blue signal B1 (see Fig. 8). Thus, as for the signal ratio B1/G2, the signal value of the green signal G2 decreases more greatly than the signal value of the blue signal B1 with increase in the blood volume. In other words, the signal ratio B1/G2 increases with increase in the blood volume.

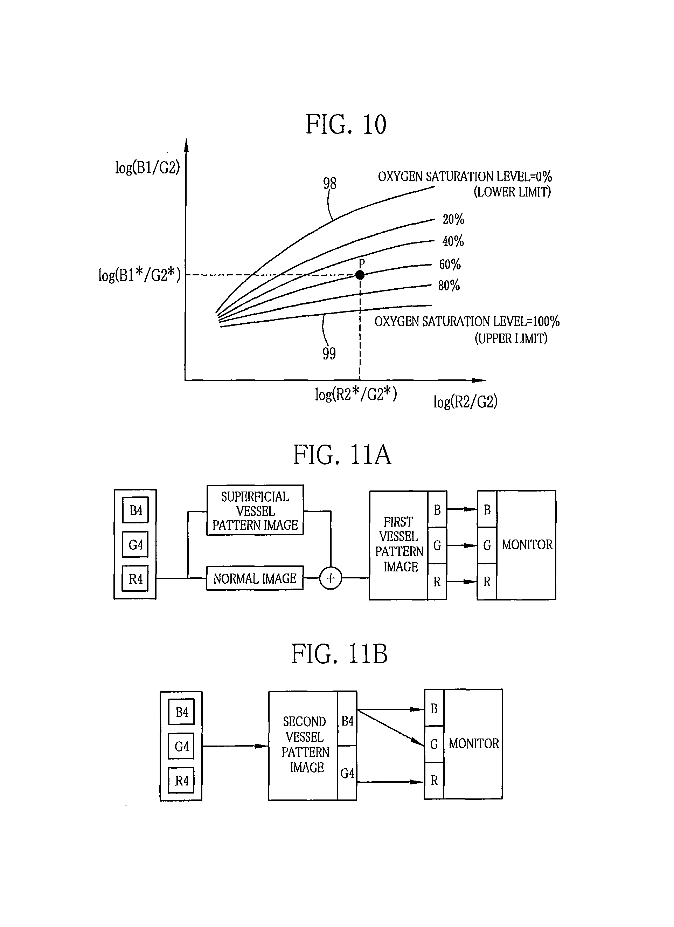

The oxygen saturation level calculator 86 calculates the oxygen saturation level of each pixel with the use of the correlations stored in the correlation memory 85 and the signal ratios B1/G2 and R2/G2 obtained by the signal ratio calculator 84. As shown in Fig. 10, a point P that corresponds to the signal ratios B1*/G2* and R2*/G2* obtained by the signal ratio calculator 84 is determined in the correlation stored in the correlation memory 85. When the point P is situated between a lower limit line 93 representing an oxygen saturation level of 0% and an upper limit line 94 representing an oxygen saturation level of 100%, the point P indicates the percentile of the oxygen saturation level. Taking Fig. 10 as an example, the point P is situated in a contour line of 60%, so the oxygen saturation level is 60%.

On the other hand, in a case where the point is out of the range between the lower limit line 98 and the upper limit line 99, if the point is situated above the lower limit line 98, the oxygen saturation level is determined to be 0%. If the point is situated below the upper limit line 99, the oxygen saturation level is determined to be 100%. Note that, in a case where the point is out of the range between the lower limit line 98 and the upper limit line 99, the oxygen saturation level of the pixel is judged to be unreliable and may not be displayed on the monitor 14.

The moving image generator 88 produces the oxygen saturation image based on the oxygen saturation level obtained by the oxygen saturation level calculator 86. The oxygen saturation moving image is produced from the produced oxygen saturation images. In the oxygen saturation moving image, for example, the entire normal moving image may be artificially colored in accordance with the degree of the oxygen saturation level. In another case, only a hypoxic area, which has the oxygen saturation level lower than a predetermined value, may be artificially colored, while the other areas may be displayed with original colors (colors used in the normal moving image).

The vessel pattern moving image processor 82 includes a first processor 92 and a second processor 93. The first processor 92 produces a series of first vessel pattern images in which superficial blood vessels are emphasized. The series of first vessel pattern images composes a first vessel pattern moving image. The second processor 93 produces a series of second vessel pattern images in which the superficial blood vessels and medium to deep blood vessels are colored differently. The series of second vessel pattern images composes a second vessel pattern moving image.

In producing the first vessel pattern image, the light intensity of the second laser beam (445 nm) is set higher than that of the third laser beam (405 nm). In other words, the pattern detection light includes green and red components at a higher rate than a blue component. In producing the second vessel pattern image, on the other hand, the light intensity of the third laser beam (405 nm) is set higher than that of the second laser beam (445 nm). In other words, the pattern detection light includes the blue component at a higher rate than the green and red components.

As shown in Fig. 11A, the first processor 92 produces the normal image and a superficial vessel pattern image by using the image signals B4, G4, and R4 obtained in the fourth frame period. The image signals B4, G4, and R4 are obtained under illumination light that includes the green and red components at the higher rate than the blue component. Thus, in the production of the normal image, the image signals B4, G4, and R4 are corrected in accordance with the ratio among the components, so as to have values equal to signals obtained under illumination light that includes the blue, green, and red components at an approximately equal rate. The normal image is produced based on the corrected image signals B4, G4, and R4.

To produce the superficial vessel pattern image, special image processing is applied to the image signals B4, G4, and R4 to extract and enhance the superficial blood vessels. The produced superficial vessel pattern image and the normal image are merged to obtain the first vessel pattern image. The B pixels, G pixels, and R pixels of the first vessel pattern image are assigned to B, G, and R channels of the monitor 14, respectively.

Note that, the special image processing includes blood vessel extraction processing using edge enhancement, frequency filtering, and a signal ratio B4/G4. For example, the spatial frequency of an image tends to increase with decrease in the thickness of a blood vessel. Thus, application of high frequency filtering processing allows extraction of a narrow superficial blood vessel. Application of low to medium frequency filtering processing allows extraction of a thick medium to deep blood vessel. Also, since the depth of a blood vessel is directly proportional to the signal ratio B4/G4, a portion having the signal ratio B4/G4 less than a predetermined value can be extracted as the superficial blood vessel. A portion having the signal ratio B4/G4 more than the predetermined value can be extracted as the medium to deep blood vessel.

As shown in Fig. 11B, the second processor 93 produces the second vessel pattern image from the two signals B4 and G4 out of the image signals obtained in the fourth frame period. The signal B4 of the second vessel pattern image is assigned to the B and G channels of the monitor 14, while the signal G4 is assigned to the R channel of the monitor 14. Therefore, the superficial blood vessels and the medium to deep blood vessels are displayed with different colors on the monitor 14. The signals B4 and G4 are obtained under illumination light that includes the blue component at the higher rate than the green and red components, so the superficial blood vessels are enhanced more than the medium to deep blood vessels.

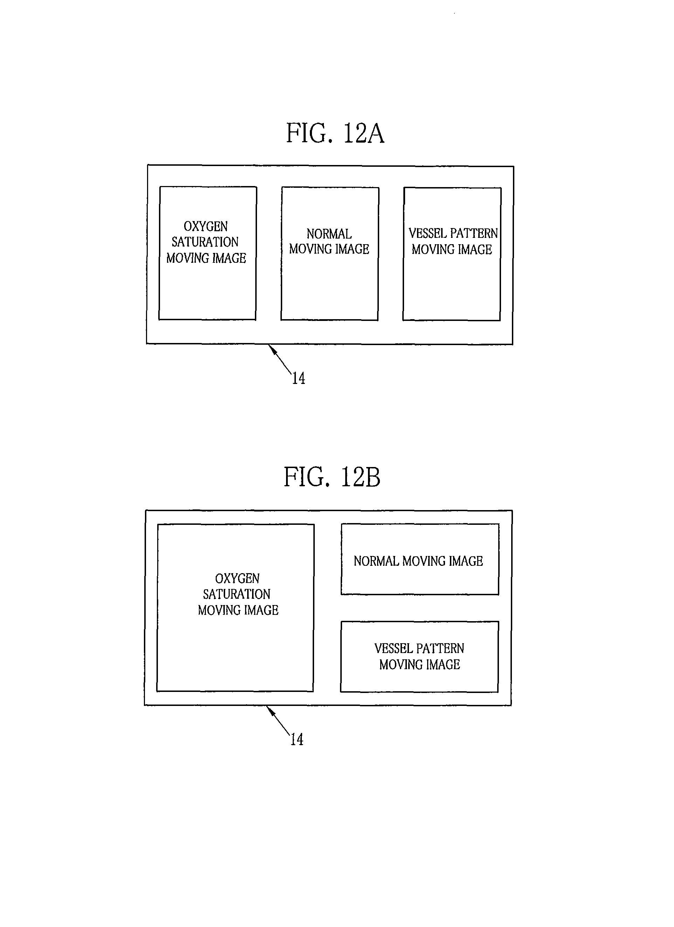

In the normal mode, the main controller 71 displays the normal moving image on the monitor 14. In the special mode, the main controller 71 displays the three moving images i.e. the oxygen saturation moving image, the normal moving image, and the vessel pattern moving image (one or both of the first and second vessel pattern moving images) on the monitor 14 in a tiled manner. The three moving images may be displayed in the same size, as shown in Fig. 12A. Alternatively, as shown in Fig. 12B, a key moving image (the oxygen saturation moving image in Fig. 12B) may be displayed larger than the other moving images (the normal moving image and the vessel pattern moving image in Fig. 12B) for the purpose of improving the visibility of the key moving image. The key moving image is set from the input device 15. The key moving image is a moving image that a doctor especially pays attention to. The oxygen saturation moving image is assigned as the key moving image in Fig. 12B, but another moving image may be assigned instead.

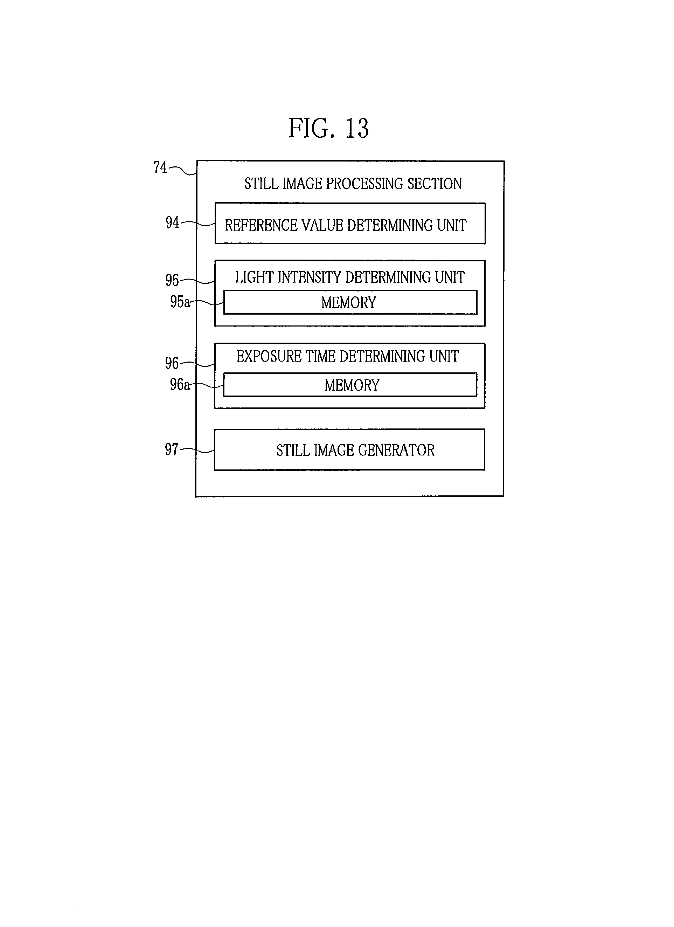

As shown in Fig. 13, the still image processing section 74 carries out the still image recording process in which when the freeze button 16a is pressed (this operation is hereinafter called "freeze"), an image captured at the time of freeze is recorded (stored) as a still image. The still image processing section 74 includes a reference value determining unit 94, a light intensity determining unit 95, an exposure time determining unit 96, and a still image generator 97. The reference value determining unit 94 calculates a reference value S from an image (immediately preceding image) that is obtained immediately before the press of the freeze button 16a. The reference value S is used for obtaining a reference exposure condition (reference light intensity and reference exposure time) to be adopted in the still image recording process.

Note that, the reference value may be any value as long as it represents an observation state at the time of freeze. For example, an average of pixel values (BGR values) of the immediately preceding image may be adopted as the reference value. The average of the pixel values of the immediately preceding image represents an exposure state at the time of freeze. An R/G ratio being a ratio between an R image and a G image of the immediately preceding image may be adopted as the reference value. This R/G ratio varies depending on the internal body portion. For example, the R/G ratio of stomach is larger than the R/G ratio of esophagus, in general.

Figs. 14 and 15 are timing charts of the still image recording process, which is carried out upon the press of the freeze button 16a in the special mode. As shown in Fig. 14, the light intensity determining unit 95 determines the light intensity (reference light intensity) P of the first and second measurement light to be emitted in the still image recording process, based on the reference value S calculated by the reference value determining unit 94. Based on the light intensity P and the ratio (La:Lb:Lc) among the light intensity La of the first and second frame periods, the light intensity Lb of the third frame period, and the light intensity Lc of the fourth frame period, which is stored advance as a fixed value on a memory 95a, the light intensity Q of the normal light and the light intensity R of the vessel detection light to be emitted in the still image recording process are calculated. The light intensity Q is obtained by multiplying the light intensity P by a ratio Lb/La. The light intensity R is obtained by multiplying the light intensity P by a ratio Lc/La. The laser sources LD1, LD2, and LD3 are controlled based on the light intensity P, Q, and R, respectively, to produce the first and second measurement light, the normal light, and the vessel detection light.

As shown in Fig. 15, the exposure time determining unit 96 determines exposure time K, L, and M of the image sensor 60 to be adopted in the first to fourth frame periods in the still image recording process. The exposure time (reference exposure time) K to be adopted in the first and second frame periods is determined based on the reference value S calculated by the reference value determining unit 94 (in consideration of the intensity of the illumination light, in fact). Then, based on the exposure time K and the ratio (Ea:Eb:Ec) among the exposure time Ea of the first and second frame periods, the exposure time Eb of the third frame period, and the exposure time Ec of the fourth frame period, which is stored advance as a fixed value on a memory 96a, the exposure time L of the third frame period and the exposure time M of the fourth frame period are calculated. The exposure time L of the third frame period is obtained by multiplying the exposure time L by a ratio Eb/Ea. The exposure time M of the fourth frame period is obtained by multiplying the exposure time K by a ratio Ec/Ea. The imaging controller 70 of the electronic endoscope 12 controls the imaging timing of the image sensor 60 based on the exposure time K, L, and M.

The still image processing section 97 produces the oxygen saturation image, the normal image, and the vessel pattern image based on the image signals obtained in the still image recording process, and outputs the produced images as the still images. A method for producing each image is the same as above, so the description thereof will be omitted. The produced oxygen saturation still image, the normal still image, and the vessel pattern still image are written to the still image storage 75a of the storage unit 75.

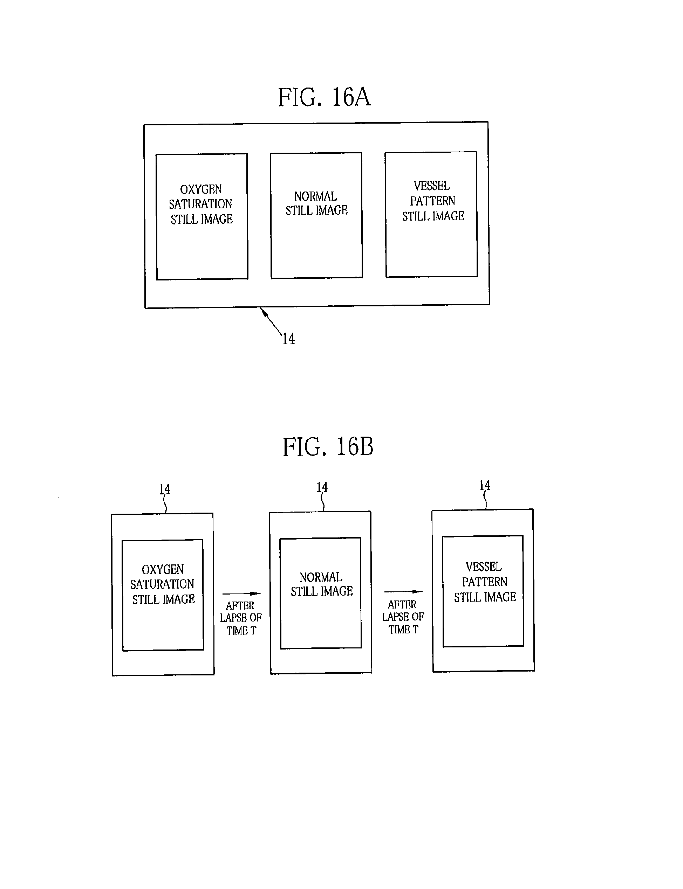

At almost the same time as the record of the still images, the three types of still images may be displayed on the monitor 14 in a tiled manner as shown in Fig. 16A, or the three types of still images may be displayed one by one on the monitor 14 in succession at intervals of regular time T like a slide show. By displaying the recorded still images on the monitor 14 immediately after the freeze, the doctor can easily check what image is to be recorded. After the display of the still images, the moving images are displayed again.

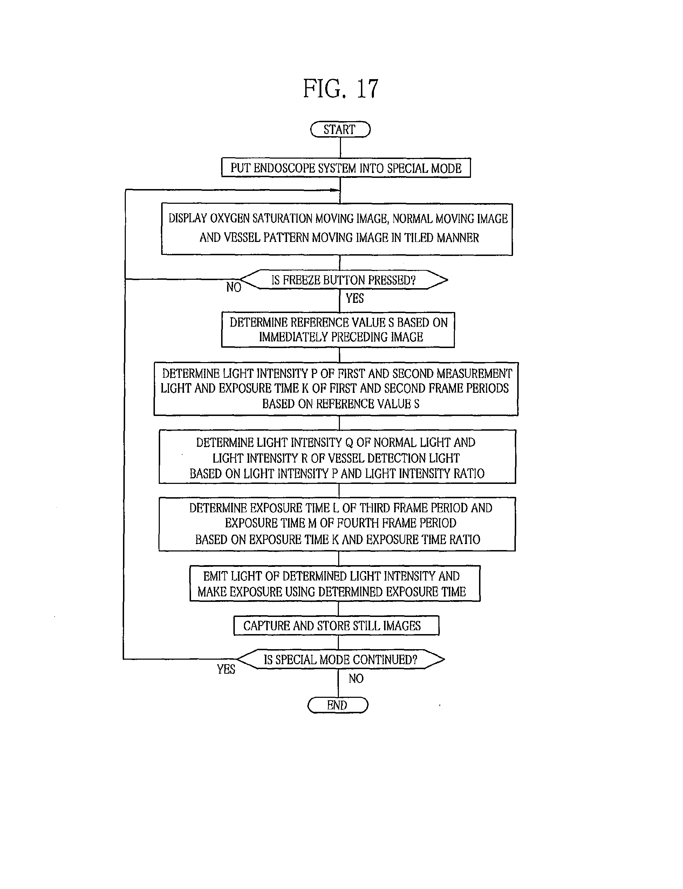

Next, the operation of the present invention will be described with referring to a flowchart of Fig. 17. The endoscope system 10 is put into the special mode by operation of the mode switch 21 of the electronic endoscope 12. Thus, the first measurement light including the first laser beam having a center wavelength of 473 nm is applied to the internal body portion in the first frame period. The second measurement light including the second laser beam having a center wavelength of 445 nm is applied to the internal body portion in the second frame period. The oxygen saturation image is produced from the image signals B1, G2, and R2 obtained in the first and second frame periods.

Next, in the third frame period, the internal body portion is imaged under the normal light. The normal image is produced from the image signals of the third frame period. In the fourth frame period, the internal body portion is imaged under the vessel detection light. The vessel pattern image is produced from the image signals of the fourth frame period.

Whenever the oxygen saturation image, the normal image, and the vessel pattern image are produced, the produced images are immediately displayed on the monitor 14. In other words, the oxygen saturation moving image, the normal moving image, and the vessel pattern moving image are displayed in a tiled manner on the monitor 14.

During the display of the moving images, when the freeze button 16a is pressed, the reference value S is calculated from the image obtained immediately before the press of the freeze button 16a. Based on the reference value S, the light intensity (reference light intensity) P of the first and second measurement light and the exposure time (reference exposure time) K of the first and second frame periods are determined. After that, the light intensity Q of the normal light is calculated by multiplying the light intensity P by the ratio Lb/La, and the light intensity R of the vessel detection light is calculated by multiplying the light intensity P by the ratio Lc/La. The exposure time L of the third frame period is calculated by multiplying the exposure time K by the ratio Eb/Ea, and the exposure time M of the fourth frame period is calculated by multiplying the exposure time K by the ratio Ec/Ea.

While the first and second measurement light, the normal light, and the vessel detection light are produced based on the calculated light intensity P, Q, and R, the first to fourth frame periods are carried out with the calculated exposure time K, L, and M. Thus, the oxygen saturation image, the normal image, and the vessel pattern image are obtained. The three types of images are recorded to the still image storage 75a of the storage unit 75 as the still images. Almost concurrently with recording, the three types of images are displayed on the monitor 14. After the display of all the three types of images, the oxygen saturation moving image, the normal moving image, and the vessel pattern moving image are displayed again on the monitor 14. The display of the moving images is continued, while the endoscope system 10 is in the special mode.

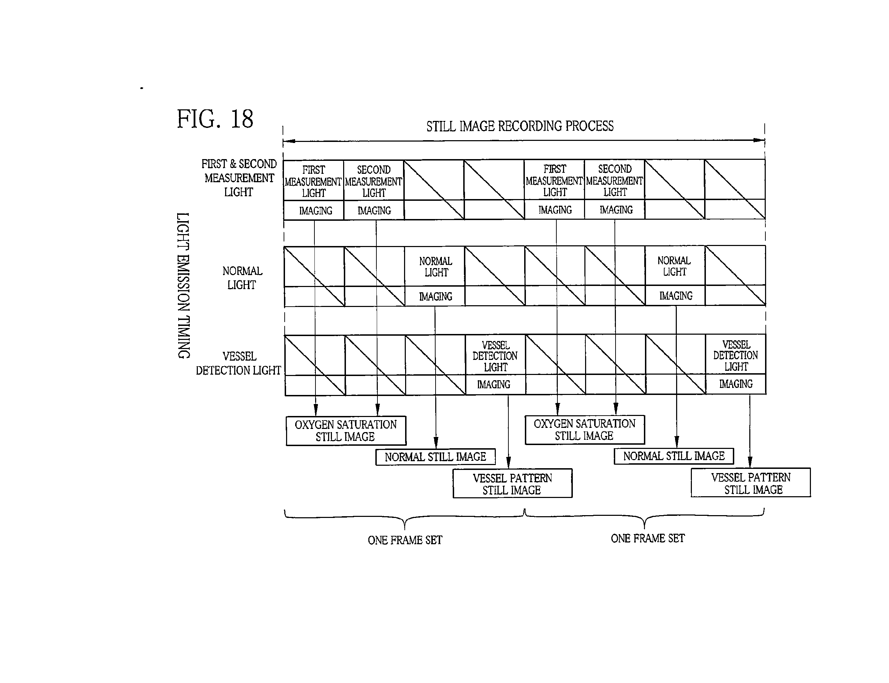

In the first embodiment, a set (frame set) of still images including the oxygen saturation image, the normal image, and the vessel pattern image is obtained upon the press of the freeze button 16a. However, as shown in Fig. 18, two frame sets may be obtained instead. Out of the two frame sets, only the frame set that has little positional deviation among first to fourth frame periods may be written to the still image storage 75a. The positional deviation among the first to fourth frame periods is judged with the use of the red signals R1 to R4, because the red signals R1 to R4 have approximately the same spectral distribution. For example, when R1/R4, R2/R4, and R3/R4 are less than a predetermined threshold value, the positional deviation among the first to fourth frame periods is judged to be little.

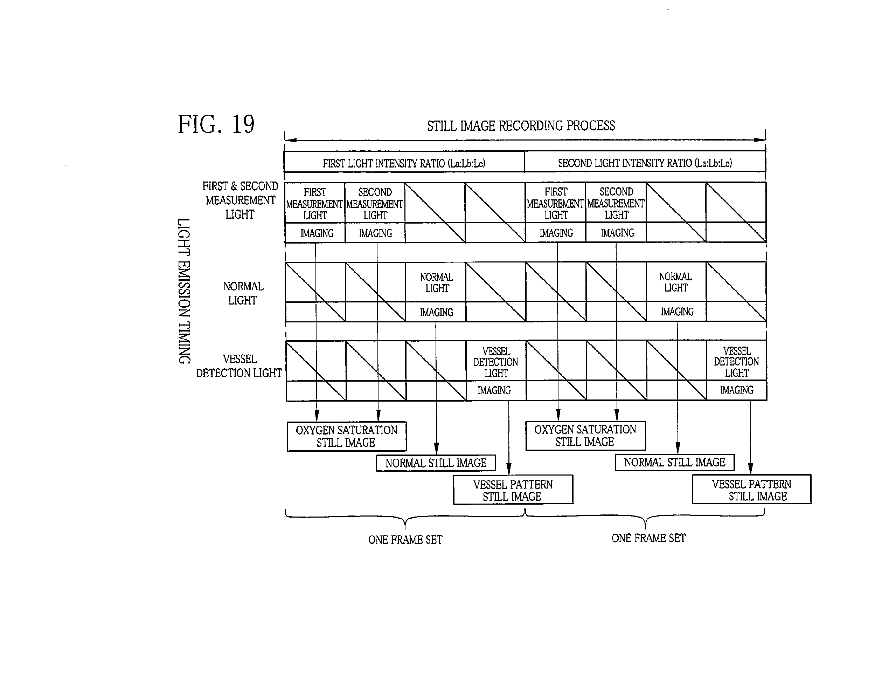

In the first embodiment, the reference value S is obtained from the immediately preceding image, and the light intensity and the exposure time are calculated from the reference value S. The illumination light is emitted with the calculated light intensity, while imaging is performed with the calculated exposure time. At this time, there may be established a plurality of light intensity ratios to be used in the still image recording process. For example, in Fig. 19, first and second light intensity ratios are used. A plurality of frame sets are sequentially obtained in accordance with each of the first and second light intensity ratios.

All the images obtained in the still image recording process may be written to the still image storage 75a as the still image, or some of the images suitable for diagnosis may be chosen manually or automatically. In the case of automatic choice, an image that satisfies certain criteria such as brightness and contrast is chosen. Note that, as for the exposure time to be used in the still image recording process, as with the light intensity ratios, a plurality of exposure time ratios may be established in advance, and a plurality of frame sets may be sequentially obtained in accordance with each of the exposure time ratios.

In the above first embodiment, the phosphor 50 is provided in the head assembly 19, but may be provided in the light source device 11. In this case, the phosphor 50 is necessarily disposed between the laser source LD2 (445 nm) and the optical fiber 25. The phosphor 50 is not necessarily disposed between the laser source LD1 (473 nm) and the optical fiber 24, and between the laser source LD3 (405 nm) and the optical fiber 26.

### (Second Embodiment)

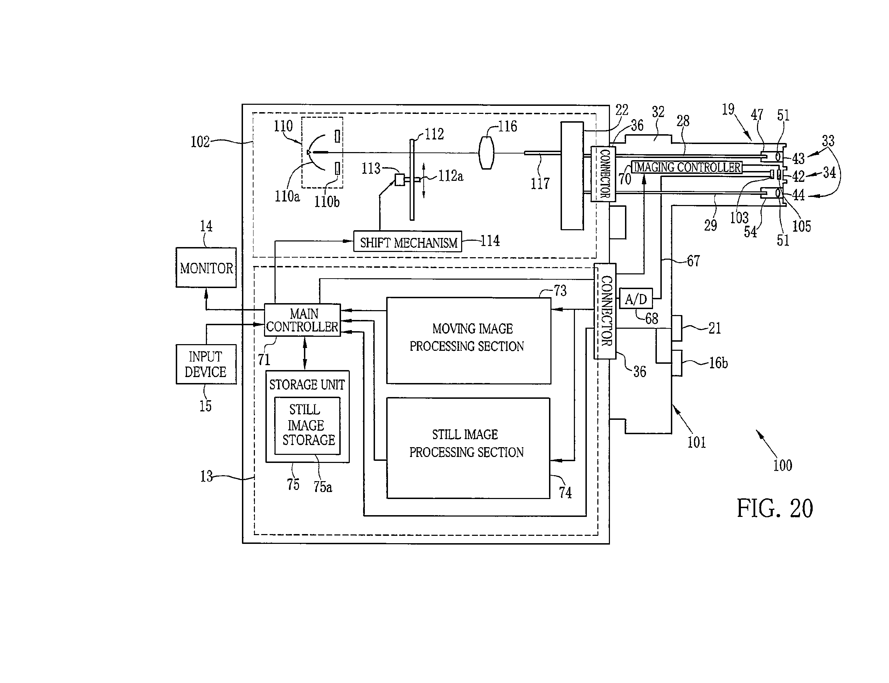

In the first embodiment, the illumination light from semiconductor light sources is used for lighting the interior of the patient's body. Instead of this, a second embodiment adopts a rotary filter method, i.e. illumination light that is extracted by a rotary filter from broadband light from a white light source such as a xenon lamp. The second embodiment uses an endoscope system 100, as shown in Fig. 20. The endoscope system 100 is identical to the endoscope system 10, except for an electronic endoscope 101 and a light source device 102. Thus, the structure of the electronic endoscope 101 and the light source device 102 and parts related thereto will be described below, and the explanation of the other parts will be omitted. Note that, the adoption of the rotary filter method sometimes makes it difficult for the endoscope system 100 to perform control of the light intensity in a short time. In such a case, the processor device 13 may not be provided with the light intensity determining unit 95.

The electronic endoscope 101 differs from the electronic endoscope 12 in terms that there is no phosphor 50 in the lighting section 33 of the head assembly 19. Thus, light from the light source device 102 is directly applied to the internal body portion through the light guides 28 and 29. Amonochrome CCD image sensor, which has no color filter in its imaging surface, is used as an image sensor 103. A mechanical shutter 105 is provided between the image sensor 103 and the imaging window 42 to regulate the exposure time. This mechanical shutter 105 is controlled by the imaging controller 70.

As for the other components, the electronic endoscope 101 has the same structure as the electronic endoscope 12. Note that, the mechanical shutter 105 is provided because a FT (frame transfer) type image sensor having no electronic shutter function is used as the image sensor 103. If an image sensor having the electronic shutter function is used as the image sensor 103, there is no need for providing the mechanical shutter 105.

The light source device 102 includes a white light source 110, a rotary filter unit 112, a motor 113, and a shift mechanism 114. The white light source 110 emits broadband light BB in wavelengths of 400 to 700 nm. The rotary filter unit 112 splits the broadband light BB emitted from the white light source 110 in accordance with a wavelength. The motor 113 is connected to a rotary shaft 112a of the rotary filter unit 112, and rotates the rotary filter unit 112 at constant speed. The shift mechanism 114 shifts the rotary filter unit 112 in a direction orthogonal to an optical axis of the broadband light BB (in a radial direction of the rotary filter unit 112).

The white light source 110 includes a main body 110a for emitting the broadband light BB and an aperture stop 110b for regulating the light amount of the broadband light BB. The main body 110a has a xenon lamp, a halogen lamp, a metal halide lamp, or the like. A light amount controller (not shown) regulates the degree of opening of the aperture stop 110b.

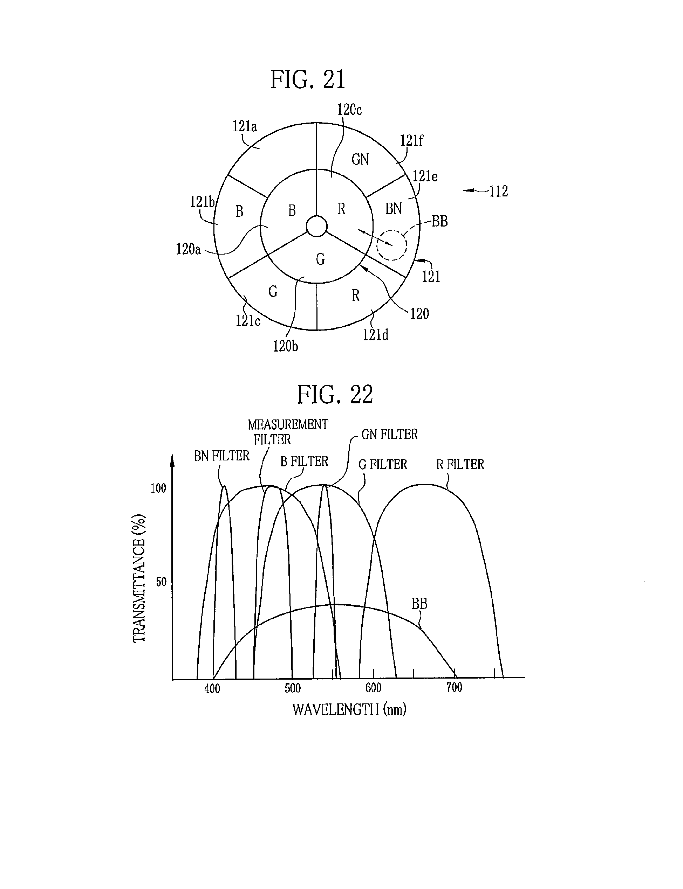

As shown in Fig. 21, the rotary filter unit 112 rotates about the rotary shaft 112a connected to the motor 113. The rotary filter unit 112 is provided with a first filter area 120 and a second filter area 121, which are disposed in this order from the side of the rotary shaft 112a in its radial direction. The first filter area 120 is set in an optical path of the broadband light BB in the normal mode. The second filter area 121 is set in the optical path of the broadband light BB in the special mode. The shift mechanism 114 performs disposition switching between the first and second filter areas 120 and 121 by a shift of the rotary filter unit 112 in its radial direction.

The first filter area 120 has a B filter 120a, a G filter 120b, and an R filter 120c each of which has the shape of a sector having a central angle of 120°. As shown in Fig. 22, the B filter 120a transmits B light in a blue wavelength band (380 to 500 nm) out of the broadband light BB. The G filter 120b transmits G light in a green wavelength band (450 to 630 nm), and the R filter 120c transmits R light in a red wavelength band (580 to 760 nm) out of the broadband light BB. Thus, by the rotation of the rotary filter unit 112, the B light, the G light, and the R light extracted from the broadband light BB are emitted in a sequential manner. The B light, the G light, and the R light enter the light guides 28 and 29 through a condenser lens 116 and an optical fiber 117.

The second filter area 121 has a measurement filter 121a, a B filter 121b, and a G filter 121c, an R filter 121d, a BN filter 121e, and a GN filter 121f each of which has the shape of a sector having a central angle of 60°. The measurement filter 121a transmits measurement light, which is in a wavelength range of 450 to 500 nm and used for measurement of the oxygen saturation level, out of the broadband light BB. The B filter 121b transmits the B light in the blue wavelength band (380 to 500 nm). The G filter 121c transmits the G light in the green wavelength band (450 to 630 nm), and the R filter 121d transmits the R light in the red wavelength band (580 to 760 nm), as with the B, G, and R filters 120a to 120c described above.

The BN filter 121e transmits blue narrowband light (BN light) having a center wavelength of 415 nm. The GN filter 121f transmits green narrowband light (GN light) having a center wavelength of 540 nm. Therefore, by the rotation of the rotary filter unit 112, the measurement light, the B light, the G light, the R light, the BN light, and the GN light are taken out in a sequential manner. These six types of light sequentially enter the light guides 28 and 29 through the condenser lens 116 and the optical fiber 117.

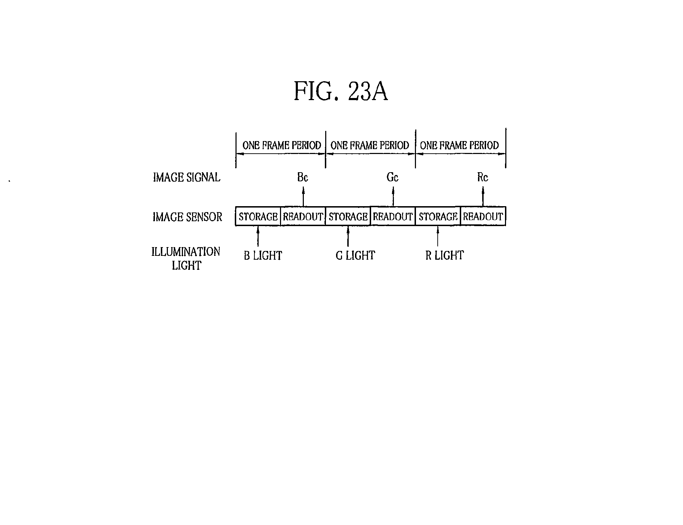

The imaging control of the endoscope system 100 is different from that of the endoscope system 10 due to the adoption of the rotary filter method. In the normal mode, as shown in Fig. 23A, the image sensor 103 captures B, G, and R colors of image light, and outputs a blue signal Bc, a green signal Gc, and a red signal Rc in a sequential manner. This operation is repeated, while the endoscope system 100 is in the normal mode. The normal moving image is produced from the blue signal Bc, the green signal Gc, and the red signal Rc.

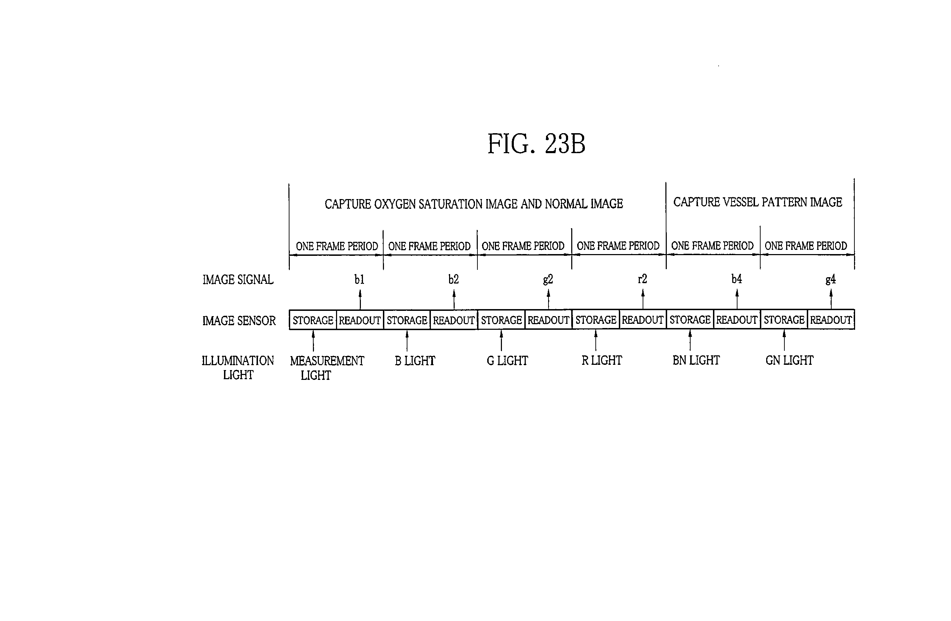

In the special mode, as shown in Fig. 23B, the image sensor 103 images the measurement light, the B light, the G light, the R light, the BN light, and the GN light, and outputs image signals in a sequential manner. This operation is repeated, while the endoscope system 100 is in the special mode. A signal b1 captured under the measurement light corresponds to the blue signal B1 of the first embodiment, because the signal b1 is based on light having a wavelength of 473 nm at which the absorption coefficient differs between the oxygenated hemoglobin and the deoxygenated hemoglobin.

A signal b2 captured under the B light corresponds to the blue signal B2 or B3 of the first embodiment, because the signal b2 is based on the B light in the blue wavelength band. A signal g2 captured under the G light corresponds to the green signal G2 or G3 of the first embodiment, because the signal g2 is based on the G light in the green wavelength band. A signal r2 captured under the R light corresponds to the red signal R2 or R3 of the first embodiment, because the signal r2 is based on the R light in the red wavelength band. A signal b4 captured under the BN light corresponds to the blue signal B4 of the first embodiment, because the signal b4 is based on light that has a blue component at a higher rate than the other components. A signal g4 captured under the GN light approximately corresponds to the green signal G4 of the first embodiment (this green signal G4 is the green signal obtained under the illumination light used in producing the second vessel pattern image), because the signal g4 is based on light that has a green component at a higher rate than the other components.

In the second embodiment, the oxygen saturation image is produced from the signals b1, g2, and r2. The normal image is produced from the signals b2, g2, and r2. The vessel pattern image is produced from the signals b4 and g4. The signals g2 and r2 are used for production of both the oxygen saturation image and the normal image. Methods for producing the oxygen saturation image, the normal image, and the vessel pattern image are the same as those of the first embodiment, so the description thereof will be omitted.

In the above embodiments, the light intensity of each type of illumination light and the exposure time of each frame period are determined based on the image captured immediately before the press of the freeze button 16b. The light intensity and the exposure time may be determined based on an image captured immediately after the press of the freeze button 16b, instead.

In the above embodiments, the press of the freeze button 16b provided in the control handle unit 16 of the electronic endoscope 12 triggers the start of the still image recording process, but a trigger is not limited to it. For example, operation of the input device 15 connected to the processor device 13 may trigger the start of the still image recording process. Note that, the oxygen saturation level is imaged in the present invention. However, an oxygenated hemoglobin index calculated by "blood volume (the sum of oxygenated hemoglobin and deoxygenated hemoglobin) x oxygen saturation level (%)" or a deoxygenated hemoglobin index calculated by "blood volume x (100 - oxygen saturation level) (%)" may be imaged instead of or in addition to the oxygen saturation level.

Although the present invention has been fully described by the way of the preferred embodiment thereof with reference to the accompanying drawings, various changes and modifications will be apparent to those having skill in this field. Therefore, unless otherwise these changes and modifications depart from the scope of the present invention, they should be construed as included therein.

## Claims

1. An endoscope system (10) comprising:
a lighting means (11, 33) for producing a plurality of types of illumination light having different wavelength bands sequentially in accordance with a plurality of types of frame periods, and applying said illumination light to an internal body portion;
an image pickup means (34) having an image sensor (60) for imaging said internal body portion, said image pickup means (34) sequentially capturing a plurality of types of frame images;
a moving image processing means (73) for producing a plurality of types of moving images, corresponding to said plurality of types of illumination light, from said plurality of types of frame images sequentially captured by said image pickup means (34);
a monitor means (14) for displaying said plurality of types of moving images;
an exposure condition determining means (95, 96) for determining an exposure condition of each of a plurality of types of still images to be recorded in a still image recording process, in accordance with said types of frame periods, with use of one of said frame images captured immediately before or immediately after a start operation of said still image recording process as a reference image;
an exposure control means (71) for controlling exposure in accordance with said exposure condition determined independently from one of said types of frame periods to another; and
a still image recording means (71) for taking out said frame images captured under control of said exposure as said still images, to perform said still image recording process.

2. The endoscope system (10) according to claim 1, wherein said exposure condition determining means (95, 96) includes:
a memory (95a, 96a) for storing correlation in said exposure condition among said plurality of types of frame images; and
a calculator for calculating said exposure condition to be used in capturing each of said still images based on said reference image and said correlation.

3. The endoscope system (10) according to claim 2, wherein said correlation is a light intensity ratio among said plurality of types of illumination light applied in said frame periods.

4. The endoscope system (10) according to claim 2, wherein said correlation is an exposure time ratio of said image sensor (60) among said frame periods.

5. The endoscope system (10) according to one of claims 1 to 4, wherein when said plurality of types of still images obtained in a sequence is referred to as a frame set, said still image recording means (71) obtains a plurality of frame sets, and calculates positional deviation among said still images in each of said frame sets, and records to still image storage (75a) said frame set whose positional deviation is less than a predetermined threshold value.

6. The endoscope system (10) according to one of claims 1 to 5, wherein after said still image recording process, all of said recorded still images are displayed on said monitor means (14) in a tiled manner.

7. The endoscope system (10) according to one of claims 1 to 5, wherein after said still image recording process, all of said recorded still images are displayed on said monitor means (14) in succession at regular intervals.

8. The endoscope system (10) according to one of claims 1 to 7, wherein said plurality of types of frame images include:
a normal image obtained under irradiation with white light;
an oxygen saturation image obtained under irradiation with measurement light having a wavelength at which a light absorption coefficient much differs between oxygenated hemoglobin and deoxygenated hemoglobin; and
a vessel pattern image obtained under irradiation with vessel detection light that has a wavelength having a high light absorption coefficient of hemoglobin and emphasizes a blood vessel of specific depth.

9. The endoscope system (10) according to one of claims 1 to 8, wherein said lighting means (11) includes a plurality of semiconductor light sources for emitting said plurality of types of illumination light.

10. The endoscope system (100) according to one of claims 1 to 8, wherein said lighting means (102) includes:
a white light source (110) for emitting broadband light;
and
wavelength selective filters (120a-120c, 121a-121f) each for taking out light of a specific wavelength from said broadband light, to produce said plurality of types of illumination light.

11. A processor device (13) of an endoscope system (10), said endoscope system (10) producing a plurality of types of illumination light having different wavelength bands sequentially in accordance with a plurality of types of frame periods and applying said illumination light to an internal body portion, while sequentially capturing a plurality of types of frame images with use of an image sensor (60), said processor device (13) comprising:
a moving image processing means (73) for producing a plurality of types of moving images, corresponding to said plurality of types of illumination light, from said plurality of types of frame images sequentially captured by said image sensor (60);
an exposure condition determining means (95, 96) for determining an exposure condition of each of a plurality of types of still images to be recorded in a still image recording process, in accordance with said types of frame periods, with use of one of said frame images captured immediately before or immediately after a start operation of said still image recording process as a reference image;
an exposure control means (71) for controlling exposure in accordance with said exposure condition determined independently from one of said types of frame periods to another; and
a still image recording means (71) for taking out said frame images captured under control of said exposure as said still images, to perform said still image recording process.

12. An exposure control method of an endoscope system (10) comprising the steps of:
producing a plurality of types of illumination light having different wavelength bands sequentially in accordance with a plurality of types of frame periods, and applying said illumination light to an internal body portion;
capturing a plurality of types of frame images sequentially with use of an image sensor (60) for imaging said internal body portion;
using as a reference image one of said frame images captured immediately before or immediately after a start operation of a still image recording process, when said start operation is performed during production of a plurality of types of moving images, corresponding to said plurality of types of illumination light, from said plurality of types of frame images sequentially captured by said image sensor (60);
determining an exposure condition of each of a plurality of types of still images to be recorded in said still image recording process, in accordance with said types of frame periods, with use of said reference image; and
controlling exposure in accordance with said exposure condition determined independently from one of said types of frame periods to another.
